# EUROPEAN PATENT SPECIFICATION

(11) **EP 1 572 284 B1**
(45) Date of publication and mention of the grant of the patent: **04.08.2010**
(21) Application number: 03799869.7
(22) Date of filing: 04.12.2003
(51) Int. Cl.: A61M 25/00

(54) **MARKED GUIDEWIRES**
MARKIERTEFÜHRUNGSDRÄHTE
FILS GUIDES MARQUES

(30) Priority: 04.12.2002 US 430835 P
(43) Date of publication of application: 14.09.2005
(73) Proprietor: LAKE REGION MANUFACTURING, INC., Chaska, Minnesota 55318 (US)
(72) Inventor: MINAR, Chris, New Prague, MN 56071 (US); RITTENOUR, Bruce, Princeton,MN 55371 (US)
(74) Representative: Carter, Stephen John
(86) International application number: PCT/US2003/038472
(87) International publication number: WO 2004/049970

(56) References cited:
- WO-A-02/47549
- WO-A1-02/47549
- GB-A- 840 725
- GB-A- 1 188 155
- US-A- 4 540 628
- US-A- 5 084 022
- US-A- 5 165 013
- US-A- 5 648 442
- US-B1- 6 306 105
- US-B1- 6 428 512
- US-B1- 6 428 512

## Description

### BACKGROUND OF THE INVENTION

This invention relates generally to the area of medical devices. More specifically, this invention relates to devices known as guidewires. Guidewires are medical devices used in numerous medical procedures. More specifically, guidewires are usually used to navigate the vasculature of the human body prior to, or in conjunction with, the placement of a separate medical device, *e.g*., a catheter, to perform a therapeutic or diagnostic procedure.

In percutaneous transluminal coronary angioplasty (PTCA) procedures a guiding catheter is first advanced in the patient's vasculature until the distal tip of the guiding catheter is seated in the ostium of a desired coronary artery. A guidewire is first advanced out of the distal end of the guiding catheter into the patient's coronary artery until the distal end of the guidewire crosses a lesion to be dilated. A dilatation catheter, having an inflatable balloon on the distal portion thereof, is advanced into the patient's coronary anatomy over the previously introduced guidewire until the balloon of the dilatation catheter is properly positioned across the lesion. Once properly positioned, the dilatation balloon is inflated with inflation fluid one or more times to a predetermined size at relatively high pressures so that the stenosis is compressed against the arterial wall and the wall expanded to open up the vascular passageway. Generally, the inflated diameter of the balloon is approximately the same diameter as the native diameter of the body lumen being dilated so as to complete the dilatation but not overly expand the artery wall. After the balloon is finally deflated, blood flow resumes through the dilated artery and the dilatation catheter and the guidewire can be removed therefrom. Much the same procedure is used in the peripheral i.e., non-coronary, vasculature, the procedure being called percutaneous transluminal angioplasty (PTA).

After such angioplasty procedures, there may be restenosis of the artery, i.e. reformation of the arterial blockage, which necessitates either another angioplasty procedure, or some other method of repairing or strengthening the dilated area. To reduce the restenosis rate of angioplasty alone and to strengthen the dilated area, physicians now normally implant an intravascular prosthesis, generally called a stent, inside the artery at the site of the lesion. Stents may also be used to repair vessels having an intimal flap or dissection or to generally strengthen a weakened section of a vessel or to maintain its patency.

Stents are usually delivered to a desired location within a vessel in a contracted condition on a balloon of a catheter, which is very similar in many respects to a balloon angioplasty catheter, and expanded within the patient's vasculature to a larger diameter by inflating the balloon. After stent deployment, the balloon is deflated, the catheter is removed and the stent is left in place within the vessel at the site of the dilated lesion or supported vessel. Thus, stents are used to keep open a stenosed vessel and to strengthen a dilated area by remaining inside the vessel. Instead of first using one catheter to dilate the body lumen and a second catheter to deploy the stent after the dilatation, the stent may be mounted on a balloon catheter and deployed at the same time the balloon is inflated to dilate the stenotic region.

In any of the above procedures the physician may want to estimate the length of the stenotic or weakened region which is to be dilated or into which a stent is to be deployed in order to assess the length of the balloon to be used for the dilatation procedure and/or the length of the stent to be deployed. Heretofore, it has been suggested to provide a variety of markers on the distal portion of the guidewire and/or catheters in order to make the length determination of stenosis. Many of these prior efforts involve providing various spacings between multiple radiopaque markers on the distal portion of the guidewire to allow the physician to make the length determination fluoroscopically with the guidewire in position within the artery and the markers traversing the stenotic region. However, due to the two dimensional nature of the fluoroscopy, these prior methods have not always been very accurate because of the orientation of the stenosis and the guidewire within the stenosis is not always suitable for an accurate length determination.

WO02/47549 discloses an elongated member, such as a wire guide, that is used in an endoscopic procedure. The elongate member includes distal and proximal portions which are coated with a polymeric material such as PET on the distal portion and PTFE on the proximal portion. Indicia are printed in ink on the PET coating.

The invention is generally directed to an improved methods and devices for observing the distance a guidewire has been inserted into a patient's vasculature and, in one aspect, measuring of non-visually observable distances within a patient's body lumen. The present invention is particularly applicable to one preferred type of guidewires, namely guidewires having lubricious, generally hydrophilic, coatings.

SUMMARY OF THE INVENTON

For any number of reasons including those discussed above, it is sometimes useful for the user of a guidewire to be able to identify the length of a guidewire which has passed into a patient's vasculature *e.g*., from a femoral artery entry site. Specifically, guidewires generally have a distal segment and a proximal segment. A distal segment of guidewire passes into a patient's vasculature and is inserted into and through the vasculature to the point where a medical procedure is to be undertaken. It is often of interest to the user to be able to identify the length of guidewire which has passed into the vasculature by reference to visually perceivable or visual indicia. Visual indicia in this context means regularly spaced or other indicia printed, sprayed, written, or otherwise impressed upon the body of the guidewire so as to be visually perceivable outside as well as inside the patient's body. The marked guidewire aspect of this invention can be deployed essentially anywhere on the guidewire body with proximal segment locations being preferred for many applications.

In another application of marked guidewires, the physician uses visual indicia to determine how far a guidewire has been inserted into a diagnostic or therapeutic catheter. Once the proximal segment marker of interest reaches the hub of the catheter, the physician then begins fluoroscopic observation of the guidewire. The physician knows that further insertion of the guidewire into the catheter causes the distal end of the guidewire to pass into the vessel. Without a visual mark the physician must begin fluoroscopic observation much earlier in the procedure so that the guidewire does not pass into the vessel without being monitored. The invention thereby reduces the patient's exposure to fluoroscopic radiation.

The invention concerns a guidewire according to claim 1 which has at least one marker or other location indicia on the distal portion of the guidewire which is observable (*e.g*. fluoroscopically) by the physician. The guidewire is positioned within the patient's body with the distal marker being placed at or adjacent to one end of the intracorporeal location to be measured and then the guidewire is repositioned so that the same distal marker is placed at, or adjacent to, the other end of the intracorporeal location. The portion of the guidewire which extends out of the patient's body moves the same distance as the distal marker is moved between the ends of the intracorporeal location to be measured and measurement of the extracorporeal movement of the guidewire is determined in order to determine the length of the intracorporeal location.

The movement of the proximal portion of the guidewire which extends out of the patient can be measured in a variety of ways. For example, ruler-like indicia, which can be seen and/or potentially felt, *e.g*. transverse ridges or grooves, can be placed on the surface of the proximal extremity of the guidewire which extends out of the patient. To make the internal measurement, the guidewire is located within the patient's body so that the distal marker on the guidewire is positioned at or adjacent to one end of the intracorporeal location to be measured. The first external position of an indicia on the proximal end of the guidewire is then referenced with respect to an external reference point, *e.g*. the proximal end of the guiding catheter adapter. When the guidewire is moved to position the distal marker at the other end of the intracorporeal location, the indicia on the proximal end of the guidewire likewise moves, and the distance it moves is the intracorporeal distance measured. The physician or other operator can determine the distance within the two intracorporeal locations by visual or manual reference to the relative movement of the ruler-like indicia to the external point of reference.

Other methods can be used to determine the distance traveled by the guidewire when changing the location of the distal marker. A wheeled distance sensing member may be pressed into engagement with the surface of the proximal end of the guidewire extending out the patient. Similarly, an electro-optical system may be utilized to measure the distance the guidewire moves into the vasculature. A wide variety of other methods may be employed to make the distance measurement. These distance measuring systems must be referenced to a suitable substrate, *e.g*. the adapter on the proximal end of the guiding catheter, so that the axial movement of the guidewire can be properly detected. To ensure that the distal position of the guidewire is not lost, it is preferred to first position the distal marker on the guidewire at the proximal intracorporeal location and then advance the guidewire distally within the body lumen until the distal marker is adjacent to the distal intracorporeal location. The reverse procedure can be employed i.e., place the distal marker at the distal end of the stenosis first and then at the proximal end of the stenosis, but in this case the guidewire must then traverse the lesion again which can be time consuming. However, by first placing the distal marker at the most distal end of the lesion and then withdrawing the guidewire proximally to move the distal marker to the proximal end of the lesion ensures that any slack present in the guidewire will be removed and thereby ensure a more accurate measurement.

The present invention thus provides improved devices for measuring the distance between two locations within a patient's vasculature, *e.g*., the length of a lesion within a blood vessel or of a weakened vascular segment. These and other advantages of the invention will become more apparent from the following detailed description of the invention when taken in conjunction with the accompanying exemplary drawings.

BRIEF DESCRIPTION OF THE DRAWINGS

FIG. 1 is a schematic, elevational view, partially in section, of a guidewire embodying features of the invention.

FIG. 2 is a transverse cross sectional view of the guidewire of FIG. 1 taken along lines 2-2.

FIG. 3 is a transverse cross sectional view of the guidewire system of FIG. 1 taken along lines 3--3.

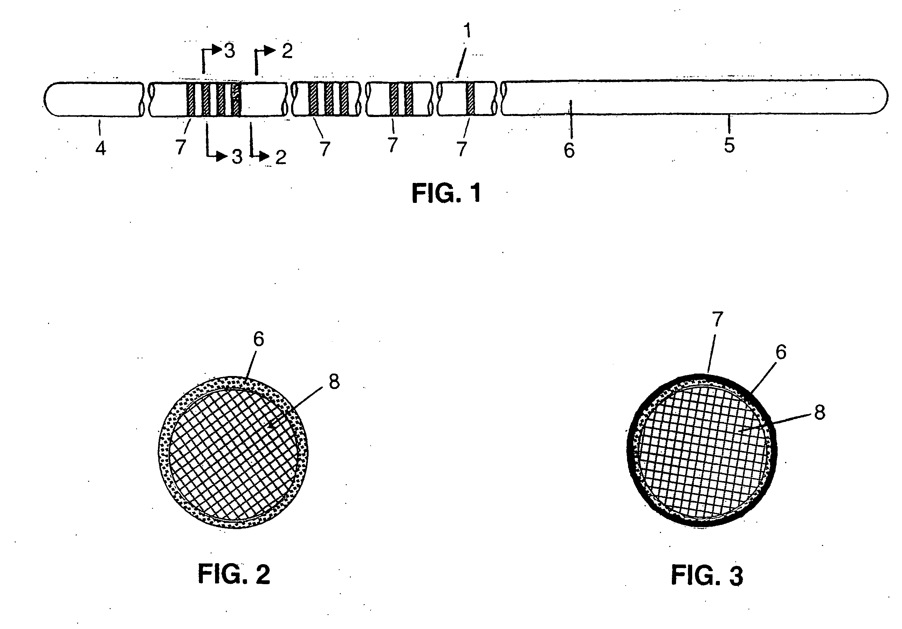

FIG. 2 shows the guidewire of FIG. 1 generally taken along the line of 2 - 2 of FIG. 1.

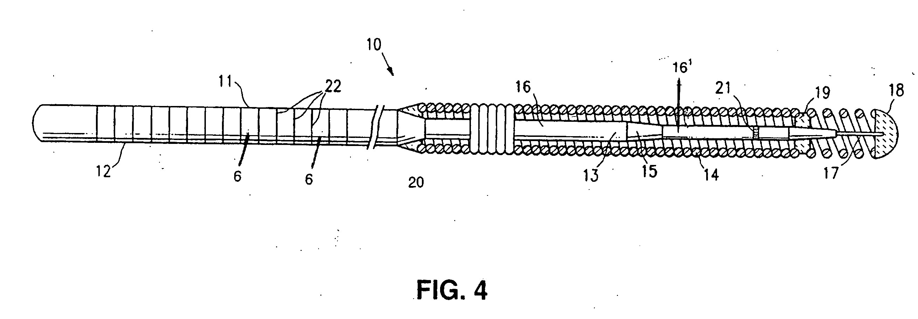

FIG. 4 is a schematic, elevational view, partially in a section of a second embodiment of the present invention.

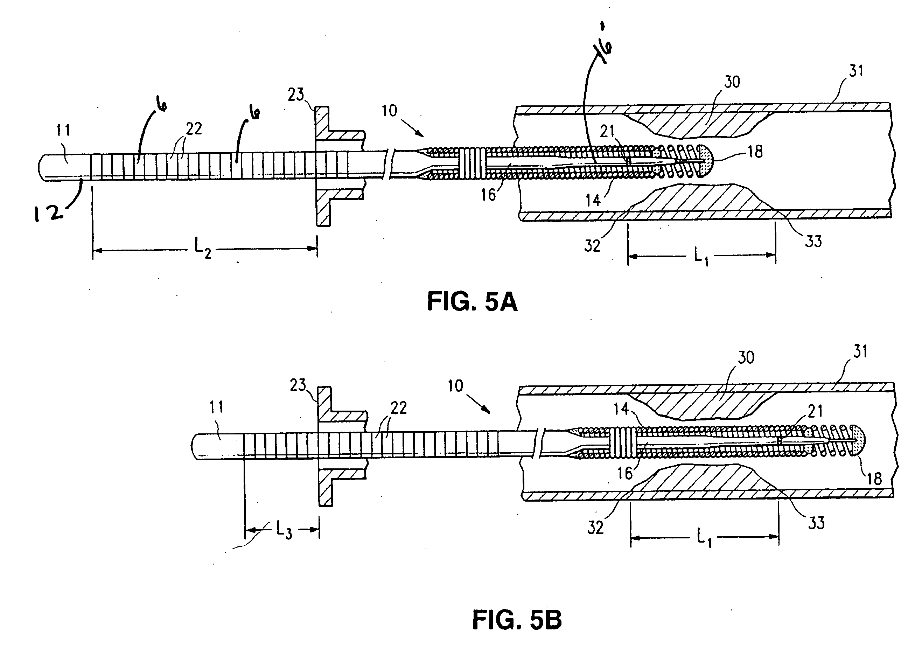

FIG. 5A is an elevational view of a guidewire within a patient's blood vessel with the distal marker adjacent to the proximal end of an arterial stenosis.

FIG. 5B is an elevational view similar to that of FIG. 5A except that the distal marker is adjacent to the distal end of an arterial stenosis.

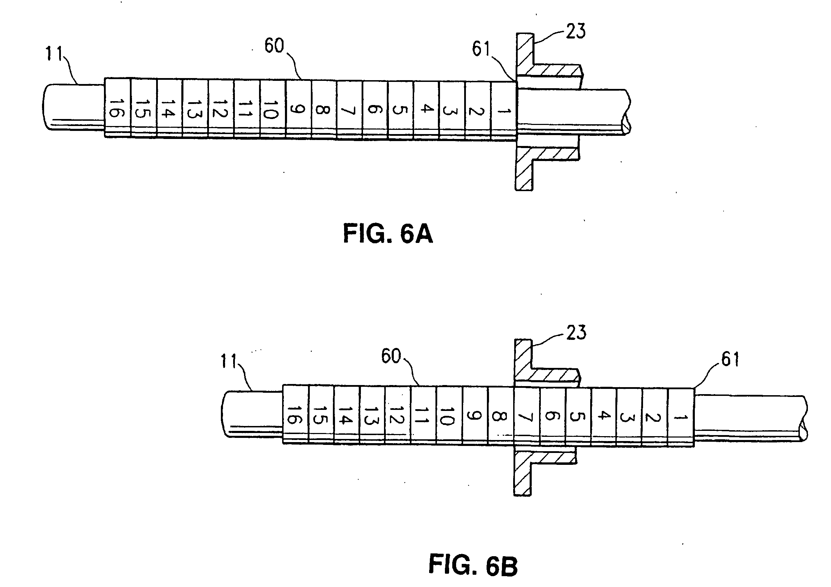

FIGS. 6A and 6B are elevational views of an alternative system for measuring the distance between the first and second intracorporeal locations wherein a slidable sheath is provided on the core of the guidewire having indicia for measuring guidewire movement.

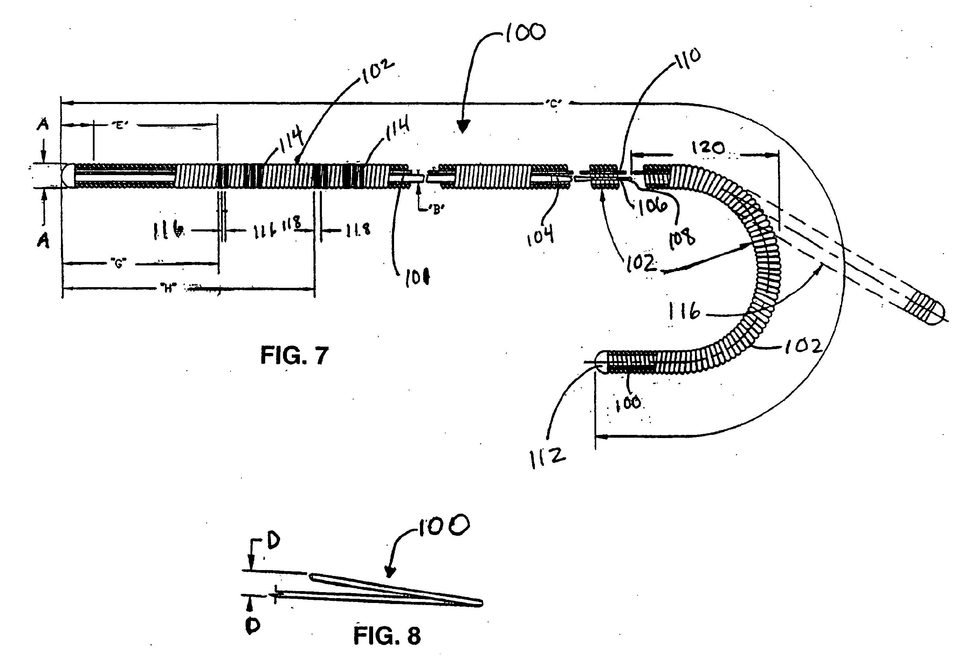

FIG. 7 is yet a further embodiment of the present invention.

FIG. 8 depicts a distal tip configuration of that guidewire shown in FIG. 7.

DETAILED DESCRIPTION OF THE INVENTION

For purposes of lubricity, commercially available guidewires tend to have a non-stick or low friction coating such as polytetrafluoroethylene (PTFE). PTFE-coated guidewires have, historically, been difficult to mark reliably and permanently so that their bodies, generally their proximal ends can be visually monitored. The present invention overcomes this difficulty in the prior art by, in one embodiment, employing a tetrafluoroethylene-based ink to create visual indicia on the body of a PTFE-coated guidewire. One skilled in this art will appreciate that other lubricious coatings within the contemplation of this invention may be employed. For example, in addition to PTFE, TFE, FEP, ETFE and numerous coatings with similar performance characteristics in the context of guidewire use may be employed. While many of the coatings suitably employed with the present invention are fluoropolymers, the invention is not limited to any particular class of polymer as its utility is believed to be widespread.

The guidewire described in U.S. 6,428,512 Anderson et al*.,* were the guidewire described therein to be PTFE-coated, is an example of where the present invention could be used. Were the present invention to be used in the context of the '512 Anderson *et al.* patent, visual or possibly tactile detection of indicia on the proximal segment of the guidewire would be deployed.

It is to be noted that the present invention relates most specifically to lubricious, hydrophobically-coated guidewires, particularly those having PTFE coatings thereon. In a further example hydrophilically-coated guidewires are discussed. In this example, a hydrophilic or hydrophilically-based ink or dye would be used to create the visual indicia on *e.g*., the proximal segment of the guidewire. Since hydrophilic coatings tend to have active functional groups exteriorly displayed or oriented, it is believed that many visually perceivable inks, dyes or other marking chemistries would likely couple to such hydrophilic coating functional groups to create permanent proximal segment guidewire markings.

FIG. 1 shows a schematic, elevational view, partially in section, of a guidewire embodying of the present invention. Guidewire 1 is an elongate guidewire-mentioned structure with its proximal end 4 being generally disposed to the left with its proximal end in section 4 being generally disposed to the left and its distal section 5 being generally disposed to the right. It is to be understood that the proximal and distal sections of a guidewire are generally understood to be from the perspective of a physician or other medical professional. Thus, the distal section segment or portion of a guidewire 5 would be generally deployed within the patient's vascular structure while the proximal end segment section or portion 4 would be that part of a guidewire handled and visually or manually contacted by the medical professional. The guidewire FIG. 1 is a hydrophobically-coated guidewire comprising a metallic core and a hydrophobic coating 6. An exemplary but by no means limiting lubricious, hydrophobic coating would be PTFE. Also shown in FIG. 1 are hydrophobic ink-based indicia 7 which are in the embodiment shown, in groups of 1, 2, 3 and 4. Clearly numerous themes and variations on the choice of indicia number, separation, width, and chemical composition, are well within the skill of one skilled in this art.

The hydrophobic-based inks or marker fluids using with the present invention have several pertinent characteristics. All usable marker fluids will "wet" the operant surface such as that of PTFE. Further, such suitable marker fluids will aggressively adhere to the hydrophobic surface or coating to which they are applied after suitable curing or subsequent curing, heating, irradiation or other bonding step(s) or adhesion. One suitable marker fluid is an aqueous TFE suspension available in various colors and various grades commercially available from GEM Gravure, Inc., of West Hanover, MA, U.S.A. A further suitable marker fluid is commercially available from Kimberly-Clark Formulabs, Neenah, Wisconsin U.S.A. It will be appreciated that the color of marker fluid chosen generally should contrast with the color of the underlying coat. Thus, for example, a green hydrophobic undercoat has been found to be suitable with a white TFE marker fluid.

Once the above marker fluids have been applied in accordance with their instruction the fluid must be cured heating to a temperate in the rage of 600 ° F. to about 950° F. Upon suitable curing, permanent indicia are created.

FIG. 4 illustrates a further embodiment of the present invention. The intracorporeal guidewire 10 generally includes an elongated core member 11 with an elongated proximal shaft section 12 and a tapered distal shaft section 13 and a helical coil 14 disposed about and secured to the tapered distal shaft section 13. The tapered distal shaft section 13 may have one or more tapered portions or sections 15 and one or more constant diameter portions 16,16'. A flat shaping ribbon 17 extends to the rounded plug 18 which is formed when the distal end of the coil 14 is welded to the distal end of the flat shaping ribbon 17. The coil 14 is also joined to the distal shaft section 13 at an intermediate location 19 and at its proximal end 20, usually by soldering or brazing. A distal radiopaque marker 21 is secured to the constant diameter portion 16 proximal to the shaping ribbon 17 so that the ribbon can be shaped without displacing the marker 21. Preferably, the marker 21 is positioned as close as possible to the intermediate location 19 so that the guidewire 10 need not be disposed too far distally when positioned adjacent to the distal end of lesion to be dilated to measure the length of the lesion.

The proximal extremity of the proximal shaft section 12 of guidewire 10 is provided with ruler-like indicia 22, such as ridges, bands, ribbons or grooves, to allow the physician or other operator to visually or possibly to manually detect how far the guidewire is axially moved with respect to a reference point such as the proximal end of an adapter 23 (shown in FIG.s 5A. and 5B.) on the proximal end of a guiding catheter (not shown) when the distal marker on the guidewire is moved from the first to the second intracorporeal location. In this embodiment the spacing between the indicia are of a standard length unit so that the physician can convert the number of indicia to a length measurement. The indicia may have suitable numbers adjacent to the indicia providing the unit measurement, e.g. mm or inches, as shown. Indicia 22 are hydrophically-based ink markings. Indicia 22 have been printed, painted, or sprayed over or on top of hydrophobic, lubricious coating 6 which has been disposed on at least a portion of proximal shaft section 12. As was noted with respect to the guidewire 1 of FIG. 1, indicia 22 do not materially change the overall diameter of guidewire 10 and thus are shown to be darker lines even though they may optionally have a substantial width and a depth or density sufficient to make them visually perceptible. It is also possible that indicia 22 may have sufficient depth or density so that they can be manually sensed or felt without changing the overall diameter or utilization of guidewire 10.

In a further aspect of the present invention to be more completely discussed below, helical coil 14 can, itself, have a hydrophobic, lubricious coating. Thus, there may be applications in which coil 14 has a hydrophobic coating thereon and for which it is desired to have visually-perceptible or manually-sensible indicia. In that embodiment, a hydrophobically-based ink, in accordance with the present invention, would be deployed on coil 14 itself.

It should be noted that the present invention could be used to place reliably dense and permanent indicia on essentially any hydrophobic coating, in essentially any configuration. Thus while bands are primarily discussed herein, longitudinal markings (lines), transverse markings, or essentially any other type of indicia are within its contemplation.

FIGS. 5A and 5B illustrate the process for measuring the length of a lesion 30 within a patient's blood vessel 31 by means of the guidewire 10 shown in FIG. 4. In FIG. 5A the guidewire 10 is shown as being positioned within the blood vessel 31 1 so that the radiopaque marker 21 is adjacent to the proximal end 32 of the lesion 30. In FIG. 5B the position of the guidewire 10 has been changed so that the radiopaque marker 21 is adjacent to the distal end 33 of the lesion 30. The distance L₁ between the proximal and distal ends 32 and 33 of lesion 30 can be determined by subtracting the distance L₂ from the distance L₃ shown at the proximal portion of the core 11 which extends out of the patient.

Another method and system for measuring the distance is shown in FIGS. 6A and 6B. In this system a slidable sleeve or other member 60 is mounted on the exterior of the proximal portion of the core member 11 which extends out of the patient. The sleeve 60 is preferably mounted to the proximal portion of the core member 11 so as to be slidable but to fit tightly enough to prevent inadvertent relative movement. When the distal marker (not shown) on the guidewire 10 is placed at or adjacent to the proximal end 32 of the lesion 30, the position of the sleeve 60 on the core member 11 is slidably adjusted until the distal end 61 of the sleeve 60 is adjacent to the proximal end of the adapter 23 on the proximal end of the guiding catheter (not shown). This locates the base measurement on the sleeve which in this case is at zero. When the position of the guidewire 10 is moved distally to shift the distal marker (not shown) to the distal end of the lesion, the sleeve 60 moves with the guidewire into the proximal opening of the adapter 23. The distance moved can be read off the indicia on sleeve 60 adjacent to the proximal end of the adapter 23.

The indicia on the proximal extremities of the guidewire generally will extend a distance of about 3 to about 40 cm to allow for the measurements of lesions throughout the patient's coronary arterial or peripheral vasculature system. With a conventional guidewire of about 175 cm, it is preferred that the markings on the proximal extremity of the core member start at a location about 40 to about 85 cm from the proximal end of the guidewire to ensure that the markings are properly located for measuring the intracorporeal length. With the use of a sheath or other slidable member such as shown in FIGS. 6A and 6B, the indicia need extend only the maximum length of the longest lesion or other intracorporeal location expected to be measured.

FIG. 7 shows a further embodiment of the present invention. In this embodiment of the invention guidewire 100 has an elongated wire core member 101 with a wound coil 102 disposed thereover. Coil 102, in this embodiment, is wound over the entire length of core wire 101. Core member 101 has a first taper 104 leading to a distally-disposed lesser diameter segment 106 and terminus 108. As is shown core member 101 terminates within coil 102 and short of the extreme distal end 112 thereof. Thus, coil 101 is coupled by means of a safety wire 110 to the extreme distal tip 112 of guidewire 100. Coil 102 comprises a wire having a PTFE (polytetrafluoroethylene) lubricious hydrophobic coat. Disposed on the proximal segment of coil 102 are a series of indicia 114 which comprise an hydrophobic-based ink. The guidewire of FIG. 7 is shown to have a "J" configuration. Shown in phantom at 116 is a slightly straightened version of the same guidewire. The user of the guidewire may, in fact, finger-straighten it in accordance with procedures well-known to those skilled in the art.

Arrows 116 show a narrow version of the hydrophobic-ink-based markers of the present invention (e.g., 1.0-2.0 mm) while arrows 118 show wider markers (e.g., 2.0-4.0 mm) in accordance with this invention. Separation distances between markers (indicia) or groups of markers will depend upon the intended guidewire use. Representative dimensions can be computed from FIG. 7 and Table 1. The longitudinal length of the "J" configuration 120 of guidewire 100 is about 2 centimeters. The guidewire shown in FIG. 7 may be substantially flat when lying upon its side. Alternatively, the "J" distal segment may not, in fact, be coplaner with the proximal segment of the guidewire as is well known in this art. This embodiment of the invention is shown in FIG. 8 which is a side view of the guidewire from the distal segment looking toward the proximal segment.

FIG. 7 illustrates various dimensions of a guidewire in accordance with the present invention by designations "A", "B", "C", "E", "G", and "H". Nominal values for those letter designations are included in Table 1. Generally speaking, the guidewire dimensions shown are those which one skilled in this art would select.

**TABLE 1**

| **"A" GUIDE DIA. +.0005 -.0010** | **"B" CORE DIA. (REF)** | **"E" UNCOATED LENGTH** | **"G" LENGTH CM ±** | **"H" LENGTH CM 1 CM** | **"C" GUIDE LENGTH IN CENTIMETERS** |
|---|---|---|---|---|---|
| .0320 | .013 | .040-.080 | 65 | 75 | 35 to 75 cm 80 to 260 cm |
| .0330 | .013 | .040-.080 | 65 | 75 | |
| .0350 | .016 | .040-.080 | 65 | 75 | |
| .0380 | .016 | .050-.090 | 65 | 75 | |

A wide variety of other means well known to those skilled in the art may be employed to detect the guidewire movement which can then be translated to the measurement of a length of a region within a patient's body. The present invention provides a basis in which such measurements can easily be made.

It will be apparent from the foregoing that, while particular forms of the invention have been illustrated and described, various modifications can be made without departing from the scope of the invention. For example, while this invention has been disclosed with reference to guidewires, other similarly-used devices, *e.g*., stylettes, and spring guides, would readily occur to one skilled in this art in light of the present disclosure. Moreover, those skilled in the art will recognize that features shown in one embodiment may be utilized in other embodiments.

## Claims

1. A guidewire (1, 10) comprising: an elongated core (11) having a proximal section (4, 12), a distal section (5, 13), and indicia, one or the other of the proximal or distal sections of the guidewire having a hydrophobic coating (6) therein, **characterized in that** the hydrophobic coating has thereon the indicia (7, 22) comprising hydrophobic ink.

2. A guidewire of claim 1 wherein a portion of the proximal section (4, 12) is hydrophobically coated and there are at least about 3 indicia (7, 22) on the proximal section.

3. A guidewire of claim 1 wherein there are a plurality of indicia (7, 22) and the plurality of indicia are uniformly spaced from each other.

4. A guidewire of claim 1 wherein there are a plurality of indicia (7, 22) and the indicia are spaced at about 0.5 to about 3 mm.

5. A guidewire of claim 1 wherein the most proximal indicia (7, 22) on the proximal extremity of the core member extends at least about 40 cm from the proximal end of the core member.

6. A guidewire according to claim 1 in which the extreme proximal end of the proximal section (4, 12) is uncoated.

7. A guidewire of claim 1 wherein the indicia (7) are visual indicia.

8. A guidewire according to claim 7 wherein the indicia (7) comprise fluoropolymers.

9. A guidewire according to claim 7 wherein the hydrophobic coating (6) consists essentially of PTFE.

10. A guidewire according to claim 7 wherein the indicia (7) comprise TFE.

11. A guidewire according to claim 7 wherein the hydrophobic coating (6) is green and the indicia (7) are white.

12. A guidewire according to claim 7 wherein the visual indicia (7) are located on the guidewire proximal section (4).

## Patentansprüche

1. Führungsdraht (1, 10), der Folgendes umfasst: einen länglichen Kern (11) mit einem proximalen Abschnitt (4, 12), einem distalen Abschnitt (5, 13) und Zeichen, wobei entweder der proximale oder der distale Abschnitt des Führungsdrahts eine hydrophobe Beschichtung (6) aufweist, **dadurch gekennzeichnet, dass** die hydrophobe Beschichtung auf diesem die Zeichen (7, 22) aufweist, die hydrophobe Tinte umfassen.

2. Führungsdraht nach Anspruch 1, worin ein Teil des proximalen Abschnitts (4, 12) hydrophob beschichtet ist und zumindest etwa 3 Zeichen (7, 22) auf dem proximalen Abschnitt vorliegen.

3. Führungsdraht nach Anspruch 1, worin eine Vielzahl an Zeichen (7, 22) vorliegt und die Vielzahl der Zeichen gleich voneinander beabstandet sind.

4. Führungsdraht nach Anspruch 1, worin eine Vielzahl an Zeichen (7, 22) vorliegt und die Zeichen etwa 0,5 bis etwa 3 mm voneinander beabstandet sind.

5. Führungsdraht nach Anspruch 1, worin sich das proximalste Zeichen (7, 22) an dem proximalen Ende des Kernelements sich zumindest etwa 40 cm von dem proximalen Ende des Kernelements erstreckt.

6. Führungsdraht nach Anspruch 1, worin das äußerste proximale Ende des proximalen Abschnitts (4, 12) unbeschichtet ist.

7. Führungsdraht nach Anspruch 1, worin die Zeichen (7) visuelle Zeichen sind.

8. Führungsdraht nach Anspruch 7, worin die Zeichen (7) Fluorpolymere umfassen.

9. Führungsdraht nach Anspruch 7, worin die hydrophobe Beschichtung (6) im Wesentlichen aus PTFE besteht.

10. Führungsdraht nach Anspruch 7, worin die Zeichen (7) TFE umfassen.

11. Führungsdraht nach Anspruch 7, worin die hydrophobe Beschichtung (6) grün ist und die Zeichen (7) weiß sind.

12. Führungsdraht nach Anspruch 7, worin die visuellen Zeichen (7) auf dem proximalen Führungsdrahtabschnitt (4) angeordnet sind.

## Revendications

1. Fil guide (1,10) comprenant: un noyau oblong (11) ayant une section proximale (4, 12), une section distale (5, 13) et des indices, l'une ou l'autre parmi les sections proximale ou distale du fil guide ayant un revêtement hydrophobe (6) à l'intérieur, **caractérisé en ce que** le revêtement hydrophobe présente sur celui-ci les indices (7, 22) comprenant de l'encre hydrophobe.

2. Fil guide selon la revendication 1, où une portion de la section proximale (4, 12) est enduite de manière hydrophobe, et il y a au moins environ 3 indices (7, 22) sur la section proximale.

3. Fil guide selon la revendication 1, où il y a plusieurs indices (7, 22), et plusieurs indices précités sont uniformément espacés les uns des autres.

4. Fil guide selon la revendication 1, où il y a plusieurs indices (7, 22), et. les indices sont espacés selon environ 0,5 à environ 3 mm.

5. Fil guide selon la revendication 1, où la plupart des indices proximaux (7, 22) sur l'extrémité proximale de l'élément de noyau s'étendent au moins sur 40 cm depuis l'extrémité proximale de l'élément de noyau.

6. Fil guide selon la revendication 1, où l'extrémité proximale extrême de la section proximale (4, 12) n'est pas revêtue.

7. Fil guide selon la revendication 1, où les indices (7) sont des indices visuels.

8. Fil guide selon la revendication 7, où les indices (7) comprennent des fluoropolymères.

9. Fil guide selon la revendication 7, où le revêtement hydrophobe (6) consiste essentiellement en PTFE.

10. Fil guide selon la revendication 7, où les indices (7) comprennent TFE.

11. Fil guide selon la revendication 7, où le revêtement hydrophobe (6) est vert, et les indices (7) sont blancs.

12. Fil guide selon la revendication 7, où les indices visuels (7) se situent sur la section proximale (4) du fil guide.
